# EUROPÄISCHE PATENTSCHRIFT

(11) **EP 0 583 689 B1**
(45) Veröffentlichungstag und Bekanntmachung des Hinweises auf die Patenterteilung: **18.07.2001**
(21) Anmeldenummer: 93112533.0
(22) Anmeldetag: 05.08.1993
(51) Int. Cl.: G01N 33/535, G01N 33/543

(54) **Immunchemisches Verfahren zum Nachweis eines Analyten**
Immunochemical method for the detection of an analyte
Procédé immunochimique pour la détection d'un analyte

(30) Priorität: 17.08.1992 DE 4227102
(43) Veröffentlichungstag der Anmeldung: 23.02.1994
(73) Patentinhaber: Dade Behring Marburg GmbH, 35001 Marburg (DE)
(72) Erfinder: Brust, Stefan, D-35041 Marburg (DE); Krupka, Udo, D-35041 Marburg (DE); Noah, Michael, D-35041 Marburg (DE); Pauly, Hans-Erwin, D-35232 Dautphetal (DE)

(56) Entgegenhaltungen:
- EP-A- 0 137 657
- EP-A- 0 306 082
- WO-A-86/07462
- US-A- 4 810 635
- JOURNAL OF CLINICAL LABORATORY ANALYSIS Bd. 5 , 1991 Seiten 197 - 205 T.KOHNO ET AL. 'Use of Inactive Beta-D-Galactosidase for Elimination of Interference by Anti-Beta-D-Galactosidase Antibodies in Immune Complex Transfer Enzyme Immunoassay for Anti-Thyroglobulin IgG in Serum Using Beta-D-Galactosidase from Escherichia coli as Label.'
- ARCHIVES OF BIOCHEMISTRY AND BIOPHYSICS Bd. 154 , 1973 Seiten 614 - 622 S.MARKLUND 'Mechanisms of the Irreversible Inactivation of Horseradish Peroxidase Caused by Hydroxymethylhydroperoxide.'

## Beschreibung

Die Erfindung betrifft ein immunchemisches Verfahren zum Nachweis eines Analyten in einer Probe eines in flüssiger Form vorliegenden biologischen Materials, bei dem die Reaktion des Analyten mit einem spezifischen Bindungspartner durch einen weiteren spezifischen Bindungspartner an den direkt oder indirekt als Markierung MeerrettichPeroxidase gekoppelt ist (Konjugat), nachgewiesen wird.

Immunologische Verfahren haben insbesondere für die klinische Diagnose aufgrund ihrer außerordentlichen Empfindlichkeit und Spezifität große Bedeutung erlangt. Bei diesen Verfahren wird in der Regel einer der immunologischen Bindungspartner markiert, d. h. mit einem Signalgeber verbunden. Gebräuchliche Markierungen sind z. B. radioaktive Isotope, fluoreszierende, phosphoreszierende oder lumineszierende Substanzen, Substanzen mit stabilen, ungepaarten Elektronen, Erythrozyten, Latexpartikel, Metallsole und Enzyme.

Innerhalb der Gruppe der zur Markierung verwendeten Enzyme ist die aus Meerrettich gewonnene Peroxidase (Donor: H₂O₂ Oxidoreductase, EC 1.11.1.7) aufgrund ihrer leichten Nachweisbarkeit und hohen Stabilität besonders geeignet. Dem Fachmann sind Reagenzien, wie z. B. bifunktionelle N-Hydroxysuccinimid-Ester, und Verfahren, wie z. B. Perjodat-Aktivierung der proteingebundenen Kohlehydrate bekannt, um Peroxidase an die immunologisch wirksamen Reaktionspartner zu koppeln. Diese Kopplungsprodukte werden als Konjugate bezeichnet, die sich insbesondere dadurch auszeichnen, daß sowohl die enzymatischen Eigenschaften der Peroxidase als auch die Rezeptoreigenschaften (z. B. Antigen oder Antikörper) weitestgehend erhalten bleiben.

Es hat sich gezeigt, daß für die überwiegende Anzahl der Proben, die mittels eines solchen Immunoassays mit Peroxidase-Konjugaten untersucht werden, richtige Ergebnisse erhalten werden. Bei einer gewissen Anzahl von Proben treten allerdings sowohl falsch positive wie auch falsch negative Befunde auf. Diese Seren werden im folgenden als Störfaktor-Seren bezeichnet.

Diese probenbezogenen Störungen des Enzymimmunoassays können durch die unterschiedlichsten Gründe hervorgerufen werden. Dem Fachmann bekannt sind Einflüsse durch in den Störfaktor-Seren enthaltene Rheumafaktoren oder heterophile Antikörper, hier insbesondere humane Anti-Maus-Antikörper. Diese Einflüsse sind in der Regel von dem verwendeten Signalgeber unabhängig.

Einige Beispiele für Störungen, die direkt durch die Enzymmarkierung bedingt sind, finden sich in der Literatur und in Patentschriften beschrieben. Kohno et al.(J. Clin. Lab. Anal., 5, pp 197-205 (1991)), beschreiben Interferenzen durch Anti-β-D-Galactosidase-Antikörper bei einem Enzymimmunoassay, der β-D-Galactosidase als Markerenzym enthält. Eliminiert wird diese Störung durch die Zugabe von gentechnisch hergestellter, enzymatisch nicht aktiver β-D-Galactosidase.

Eine weitere Möglichkeit offenbart die EP 0 209 155: falsche Resultate, die durch Wechselwirkungen von Serumbestandteilen mit Kohlehydratresten von Markerenzymen, insbesondere Peroxidase, hervorgerufen werden, können durch oxidative Entfernung dieser Zucker eliminiert werden. Nachteilig für die Funktion des Enzymimmunoassays ist bei diesem Verfahren die Herabsetzung der Enzymreaktivität um etwa 50% sowie die Aggregation des oxidierten Enzyms bzw. Konjugates. Außerdem werden Interferenzen, die im Proteinteil der Peroxidase ihre Ursache haben, durch diese Methode nicht vermieden.

Einen anderen Ansatz offenbart die Internationale Patentanmeldung PCT/GB86/00324 (WO 86/ 07462): Störungen, die durch die Anwesenheit eines Enzyminhibitors in einem Testverfahren, beinhaltend die Inkubation einer Körperflüssigkeitsprobe mit einem enzymmarkierten Reagenz, hervorgerufen werden, können demnach durch Zugabe von Apomeerrettichperoxidase, einem sauren Meerrettichperoxidaseisoenzym oder einem carboxymethylierten Meerrettichperoxidaseisoenzym als Korrekturfaktor beseitigt werden.

Einen wiederum anderen Ansatz verfolgt das US-Patent Nr. 4 810 635: hierin wird vorgeschlagen, die in einem bestimmten Testsystem (ARIS) beobachteten die Reaktivität der im Test eingesetzten Apoglucoseoxidase inhibierenden_Störfaktoren (Inhibitoren) zu beseitigen.

Gegenstand der Erfindung ist daher ein immunchemisches Verfahren zum Nachweis eines Analyten in einer Probe eines in flüssiger Form vorliegenden biologischen Materials bei dem die Reaktion des Analyten mit einem spezifischen Bindungspartner durch einen weiteren spezifischen Bindungspartner, an den direkt oder indirekt als Markierung Meerrettich Peroxidase gekoppelt ist (Konjugat), nachgewiesen wird, wobei der Reaktionsansatz zusätzlich hydroxymethylhydroperoxid-inaktivierte Peroxidase enthält.

Vorteilhaft ist dabei ein solches Verfahren, wobei die hydroxymethylhydroperoxid-inaktivierte Peroxidase vor der Markierungs-Peroxidase zum Testansatz zugegeben wird.

Vorteilhaft ist ferner ein solches Verfahren, wobei die hydroxymethylhydroperoxid-inaktivierte Peroxidase gleichzeitig mit der Markierungsperoxidase zugegeben wird.

Besonders vorteilhaft ist ein solches Verfahren, wobei die hydroxymethylhydroperoxid-inaktivierte Peroxidase vor Zugabe der Markierungsperoxidase wieder entfernt wird.

Vorteilhafterweise wird die hydroxymethylhydroperoxid-inaktivierte Peroxidase in einer solchen Menge zugegeben, daß sie im Reaktionsansatz in einer Konzentration von 0.002 bis 10 mg/ml vorliegt.

Bevorzugterweise liegt die hydroxymethylhydroperoxid-inaktivierte Peroxidase in einem Konzentrationsbereich von 0.02 bis 5 mg/ml vor, besonders bevorzugterweise in einem Konzentrationsbereich von 0.2 bis 2 mg/ml.

Bevorzugt als Nachweisverfahren ist ein Festphasenimmunoassay, ganz besonders bevorzugt ist dabei als Festphase eine Mikrotitrationsplatte.

Gegenstand der Erfindung ist ferner die Verwendung von hydroxymethylhydroperoxid-inaktivierter Peroxidase in einem immunchemischen Nachweisverfahren.

Nachweis im Sinne dieser Erfindung bedeutet sowohl qualitativer Nachweis als auch quantitative Bestimmung.

Der Erfindung liegt daher ein immunchemisches Verfahren zugrunde, Reagenzien zu entwickeln, die Wechselwirkungen von Serumstörfaktoren mit dem Markerenzym Peroxidase vermeiden, ohne die o.g. Nachteile in Kauf zu nehmen.

Überraschenderweise wurde gefunden, daß, nach Zugabe von hydroxymethylhydroperoxid-inaktivierter Peroxidase zum Konjugat Störfaktorproben, die Interferenzen mit Peroxidase-Konjugaten zeigen, richtige Resultate liefern.

Vorteilhaft ist die einfache Zugänglichkeit des erfindungsgemäßen Mittels, d. h. man kann von vorhandener, in großen Mengen zugänglicher und kostengünstiger Meerrettich-Peroxidase ausgehen, "inaktive Peroxidase" kann daraus durch die Behandlung mit Hydroxymethylhydroxiperoxid (Marklund, Arch. Biochem. Biophys. 154, S. 614 - 622 (1973) hergestellt werden. Vorteilhaft ist, daß man dabei ein inaktives Protein erhält, das dem nativen Protein bezüglich seines antigenen Charakters (Tertiär-, Quartärstruktur, Glykosilierung) sehr ähnlich ist.

Ohne damit die Erfindung auf eine bestimmte Wirkungsweise einzuschränken, ist eine mögliche Erklärung für die Wirksamkeit der hydroxymethylhydroperoxid-inaktivierten Peroxidase " die, daß sie mögliche Störfaktoren abfängt, die somit nicht an die Peroxidase binden können.

Einen vergleichbaren Effekt hat die Zugabe von Rezeptoren, die gegen Zuckerreste oder Epitope der Peroxidase gerichtet sind, nämlich, daß unspezifische Reaktionen mit Störfaktorproben unterbunden werden. Als Rezeptor kann hierbei beispielsweise ein mit der Peroxidase reagierender, monovalenter Antikörper oder ein Lectin dienen, wobei die Reaktion mit den Rezeptoren nicht zu einer Präzipitation oder Agglutination führt.

Bevorzugt sind solche Antikörper oder Lectine, die die enzymatische Aktivität der Peroxidase nicht beeinträchtigen.

Die erfindungsgemäß zu verwendender Reagenzien zeichnen sich insbesondere dadurch aus, daß sie weder die enzymatische Aktivität der Peroxidase beeinflussen noch durch Aggregation das immunologische Verhalten des Konjugates beeinträchtigen.

Als Pufferzusatz können die erfindungsgemäßen Reagenzien in einer Vielzahl von Immunoassays mit Peroxidase-Konjugaten als essentieller Testbestandteil in der Human- und Veterinär-Diagnostik Verwendung finden. Als Beispiele sollen angeführt werden ein- oder mehrstufige Tests zum Nachweis von Antikörpern verschiedener Immunglobulin-Klassen gegen Strukturmerkmale von Viren (z. B. Viren der Hepatitis A, B, C sowie verschiedener HIV-Typen), von bakteriellen und parasitären Erregern sowie von allergischen Erkrankungen. Weitere Beispiele sind der Nachweis in ein- oder mehrstufigen Nachweisverfahren von Krankheitserregern wie Viren (z. B. Hepatitis B-Virus), Bakterien, Parasiten oder Allergenen wie auch von Markern von Krankheiten (z. B. Tumormarker).

Die folgenden Beispiele erläutern den erfindungsgemäßen Gegenstand, ohne ihn einzuschränken.

### Beispiel 1:

a) Herstellung enzymatisch inaktiver Peroxidase
   Die Herstellung der enzymatisch irreversibel inaktivierter Peroxidase erfolgte durch Behandlung kommerzieller Peroxidase mit Hydroxymethylhydroperoxid nach Marklund (Arch. Biochem. Biophys. 154, S. 614-622 (1973)). Die Ausbeute betrug nach Proteinbestimmung 60 %. Die inaktive Peroxidase wurde in einer Konzentration von 20-30 g/l in 50 mM Tris, pH 8.0 bei -20 °C gelagert.
b) Sequentiell kompetitiver Anti-HIV 1 Nachweis
   Die mit gereingtem, inaktiviertem HIV 1-Lysat beschichteten Mikrotitrationsplatten der kommerziellen Testpackung "Enzygnost Anti-HIV 1" (Behringwerke AG, Marburg, FRG, Produktnummer OUVB) wurden mit 100 *µ*l der zu untersuchenden Probe versetzt und 60 min inkubiert. Danach erfolgt ohne Waschschritt die Zugabe von 25 *µ*l Anti-HIV(human)/Peroxidase-Konjugat (Behringwerke AG, Marburg, FRG, Produktnummer OWSB) mit entsprechend Beispiel 1 a) hergestellter, inaktiver Peroxidase (Konzentration in Konjugat: 1 mg/ml). Nach weiteren 30 min wird abgesaugt, gewaschen und die gebundene Peroxidase mittels des kommerziell erhältlichen Chromogensystems (Behringwerke AG, Marburg, FRG, Produktnummer OUVF/OUVG) nachgewiesen. Danach wurde nach 30 min Substratreaktionszeit mit verd. Schwefelsäure die Reaktion gestoppt und bei 450 nm das umgesetzte Chromogen detektiert. Als reaktiv wurden alle Proben angesehen, die Extinktionen unterhalb des Grenzwertes (Extinktion der negativen Kontrolle * 0.5) aufwiesen.

Die bei der Testung von Anti-HIV positiven und negativen Seren sowie von Störfaktor-Seren erhaltenen Extinktionen sind in Tabelle 1 zusammengefaßt. Erkennbar ist, daß die Leistungsfähigkeit des Tests für die richtige Erkennung "normaler" Anti-HIV positiver und negativer Proben durch den erfindungsgemäßen Zusatz nicht beeinflußt wird. Anti HIV 1 positive Störfaktor-Seren, die ohne den Zusatz von inaktiver Peroxidase falsch-negativ reagieren, werden im erfindungsgemäßen ELISA richtig-positiv gefunden.

Störfaktorhaltige Anti-HIV negative Seren ergeben in dem Test ohne erfindungsgemäßen Zusatz Extinktionswerte zum Teil über dem Meßbereich. In dem erfindungsgemäßen ELISA liegen die Extinktionswerte dieser Seren im Bereich der normalen negativen Seren.

**Tabelle 1:**

| **Probe** | **Konjugat ohne erfindungsgem. Zusatz** | **Konjugat mit erfindungsgem. Zusatz** |
|---|---|---|
| **Grenzwert** | 0,574 | 0,579 |
| **Kontrollserum,** | 1,189 | 1,121 |
| **negativ** | 1,109 | 1,193 |
| **Kontrollserum,** | **0,007** | **0,011** |
| **positiv** | **0,009** | **0,015** |
| **Anti-HIV neg. Seren** | | |
| | | |
| # 12 | 1,090 | 1,258 |
| # 45 | 1,247 | 1,147 |
| # 216 | 1,198 | 1,111 |
| **Anti-HIV 1 pos. Seren** | | |
| | | |
| WT 17 | **0,015** | **0,005** |
| WT 19 | **0,058** | **0,051** |
| WT 22 | **0,021** | **0,009** |
| **Störfaktor-Seren** | | |
| | | |
| **Anti-HIV 1 positiv** | | |
| # 40 | 1,864 | **0,032** |
| # 42 | > 2,500 | **0,060** |
| # 43 | > 2,500 | **0,056** |
| # 44 | 1,023 | **0,026** |
| # 51 | > 2,500 | **0,053** |
| **Störfaktor- Seren** | | |
| | | |
| **Anti-HIV negativ** | | |
| | | |
| # 00181 | > 2,500 | 1,029 |
| # 00202 | > 2,500 | 1,254 |
| Reaktive Proben sind fett gedruckt. | | |

### Beispiel 2:

### Halbmonoklonaler Sandwichassay zum Nachweis von HBsAg

100 *µ*l des zu untersuchenden Serums wurden in einer mit Anti-HBs (Schaf) beschichteten Mikrotitrationsplatte ("Enzygnost HBsAg monoclonal", Behringwerke AG, Marburg, FRG, Produktnummer OWNE) mit 25 *µ*l Anti-HBs (Maus)/Peroxidase-Konjugat (Behringwerke AG, Marburg, FRG, Produktnummer OWNF) für 90 min inkubiert. Ein Teil der Untersuchungen wurde ohne erfindungsgemäßen Zusatz, ein anderer Teil mit Zusatz von inaktiver Peroxidase (1 mg/ml) und ein weiterer unter Hinzufügen des Lectins Tetragonolobus purpureas (Sigma, Deisenhofen, FRG) in einer Konzentration von 1 mg/ml zum Konjugat durchgeführt. Anschließend wurde gewaschen und, wie in Beispiel 1 erläutert, die gebundene Enzymaktivität bestimmt. Als reaktiv wurden alle Proben erachtet, deren Extinktion mindestens 0.050 E über dem Mittelwert der negativen Kontrollen lag.

Die Resultate der Experimente sind in Tabelle 2 zusammengefaßt. Während die erfindungsgemäßen Zusätze die Richtigkeit der Ergebnisse bei "normalen" HBsAg positiven und negativen Proben nicht beeinflussen, werden HBsAg negative störfaktorhaltige Seren die ohne erfindungsgemäßen Konjugatzusatz falsch positiv reagieren, sowohl durch die Zugabe von inaktiver Peroxidase als auch durch das Lectin Tetragonolobus purpureas richtig negativ gefunden.

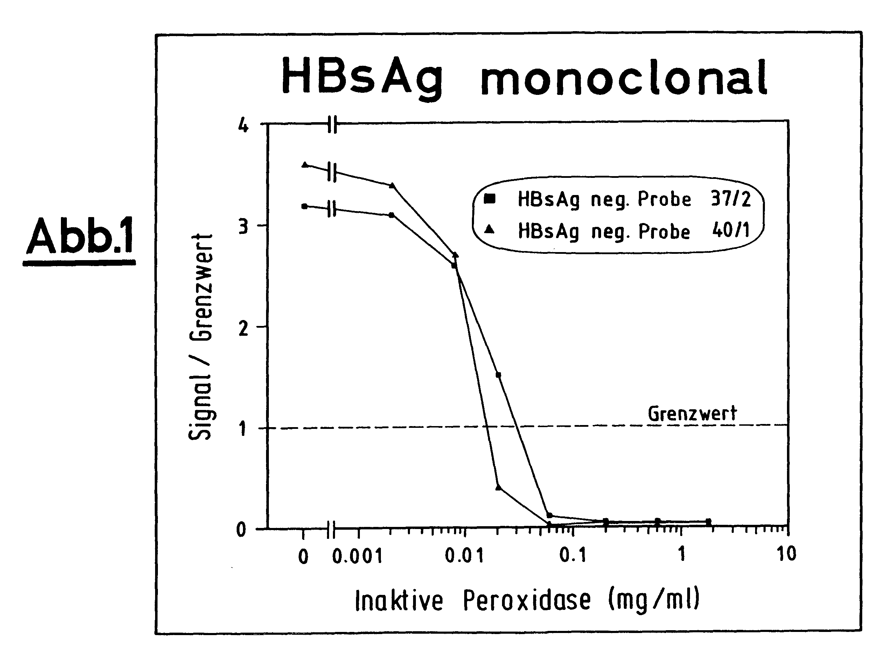

In Abb. 1 ist das Signalverhalten von zwei HBsAg neg. Proben im Verhältnis zu der dem Testansatz hinzugefügten Menge an inaktiver Peroxidase dargestellt.

**Tabelle 2:**

| **Probe** | **Konjugat ohne erfindungsgemäßem Zusatz** | **Konjugat mit erfindungsgemäßem Zusatz** | |
|---|---|---|---|
| | | **inakt. Peroxidase** | **Lectin** |
| **Grenzwert** | 0,072 | 0,062 | 0,082 |
| **Kontrollserum** | 0,026 | 0,010 | 0,031 |
| **negativ** | 0,017 | 0,014 | 0,033 |
| **Kontrollserum** | **1,266** | **1,155** | **1,019** |
| **positiv** | **1,279** | **1,241** | **0,994** |
| **HBsAg neg. Seren** | | | |
| | | | |
| # 20 | 0,025 | 0,016 | 0,022 |
| # 21 | 0,028 | 0,014 | 0,036 |
| # 24 | 0,031 | 0,011 | 0,012 |
| **HBsAg pos. Seren** | | | |
| | | | |
| # 88 | **> 2,500** | **> 2,500** | **> 2,500** |
| # 107 | **> 2,500** | **> 2,500** | **> 2,500** |
| # 209 | **> 2,500** | **> 2,500** | **> 2,500** |
| **Störfaktor-Seren** | | | |
| | | | |
| **HBsAg negativ** | | | |
| | | | |
| M 487 | **0,167** | 0,018 | 0,028 |
| M 640 | **0,139** | 0,022 | 0,039 |
| M 642 | **0,187** | 0,009 | 0,023 |
| M 674 | **0,256** | 0,016 | 0,013 |
| M 687 | **0,272** | 0,006 | 0,029 |
| Reaktive Proben sind fett gedruckt. | | | |

## Patentansprüche

1. Immunchemisches Verfahren zum Nachweis eines Analyten in einer Probe eines in flüssiger Form vorliegenden biologischen Materials, bei dem die Reaktion des Analyten mit einem spezifischen Bindungspartner durch einen weiteren spezifischen Bindungspartner, an den direkt oder indirekt als Markierung Meerrettichperoxidase gekoppelt ist (Konjugat), nachgewiesen wird, dadurch gekennzeichnet, daß der Reaktionsansatz zusätzlich hydroxymethylhydroperoxid-inaktivierte Peroxidase enthält.

2. Verfahren nach Anspruch 1, dadurch gekennzeichnet, daß es sich um einen Festphasenenzymimmunoassay handelt.

3. Verfahren nach Anspruch 1, dadurch gekennzeichnet, daß die hydroxymethylhydroperoxid-inaktivierte Peroxidase vor der Markierungs-Peroxidase zum Testansatz zugegeben wird.

4. Verfahren nach Anspruch 1, dadurch gekennzeichnet, daß die hydroxymethylhydroperoxid-inaktivierte Peroxidase gleichzeitig mit der Markierungsperoxidase zugegeben wird.

5. Verfahren nach Anspruch 1, dadurch gekennzeichnet, daß die hydroxymethylhydroperoxid-inaktivierte Peroxidase vor Zugabe der Markierungsperoxidase wieder entfernt wird.

6. Verfahren nach mindestens einem der Ansprüche 1 - 4, dadurch gekennzeichnet, daß die hydroxymethylhydroperoxid-inaktivierte Peroxidase in einer Konzentration von 0.002 bis 10 mg/ml Testansatz vorliegt.

7. Verfahren nach Anspruch 1, dadurch gekennzeichnet, daß die hydroxymethylhydroperoxid-inaktivierte Peroxidase in einem Konzentrationsbereich von 0.02 bis 5 mg/ml vorliegt.

8. Verfahren nach Anspruch 1, dadurch gekennzeichnet, daß die hydroxymethylhydroperoxid-inaktivierte Peroxidase in einem Konzentrationsbereich von 0.2 bis 2 mg/ml vorliegt.

9. Verwendung von hydroxymethylhydroperoxid-inaktivierter Peroxidase in immunchemischen Nachweisverfahren.

10. Verwendung von hydroxymethylhydroperoxid-inaktivierter Peroxidase in einem immunchemischen Diagnostikum zum Nachweis eines Analyten in einer Probe eines in flüssiger Form vorliegenden biologischen Materials.

11. Verfahren gemäß Anspruch 2, dadurch gekennzeichnet, daß die Festphase eine Mikrotitrationsplatte ist.

## Claims

1. An immunochemical method for detecting an analyte in a sample of a biological material, which is present in fluid form, in which method the reaction of the analyte with a specific binding partner is detected by a further specific binding partner to which horseradish peroxidase is coupled (conjugate) directly or indirectly as the label, wherein the reaction mixture additionally contains hydroxymethylhydroperoxide-inactivated peroxidase.

2. The method as claimed in claim 1, which is a solid-phase enzyme immunoassay.

3. The method as claimed in claim 1, wherein the hydroxymethylhydroperoxide-inactivated peroxidase is added to the test mixture before the labeling peroxidase.

4. The method as claimed in claim 1, wherein the hydroxymethylhydroperoxide-inactivated peroxidase is added at the same time as the labeling peroxidase.

5. The method as claimed in claim 1, wherein the hydroxymethylhydroperoxide-inactivated peroxidase is removed again before the addition of the labeling peroxidase.

6. The method as claimed in at least one of claims 1-4, wherein the hydroxymethylhydroperoxide-inactivated peroxidase is present at a concentration of 0.002 to 10 mg/ml of test mixture.

7. The method as claimed in claim 1, wherein the hydroxymethylhydroperoxide-inactivated peroxidase is present in a concentration range of 0.02 to 5 mg/ml.

8. The method as claimed in claim 1, wherein the hydroxymethylhydroperoxide-inactivated peroxidase is present in a concentration range of 0.2 to 2 mg/ml.

9. Use of hydroxymethylhydroperoxide-inactivated peroxidase in immunochemical detection methods.

10. Use of hydroxymethylhydroperoxide-inactivated peroxidase in an immunochemical diagnostic for detecting an analyte in a sample of a biological material which is present in fluid form.

11. The method as claimed in claim 2, wherein the solid phase is a microtitration plate.

## Revendications

1. Procédé immunochimique pour la détection d'un analyte dans un échantillon d'un matériel biologique présent sous forme liquide, dans lequel la réaction de l'analyte avec un partenaire de liaison spécifique est détectée au moyen d'un autre partenaire de liaison spécifique auquel est directement ou indirectement couplée, en tant que marqueur, de la peroxydase de raifort (conjugué), caractérisé en ce que le mélange réactionnel contient en outre de la peroxydase inactivée par de l'hydroxyméthylhydroperoxyde.

2. Procédé selon la revendication 1, caractérisé en ce qu'il s'agit d'un essai immunoenzymatique en phase solide.

3. Procédé selon la revendication 1, caractérisé en ce que la peroxydase inactivée par l'hydroxyméthylhydroperoxyde est ajoutée au mélange d'essai avant la peroxydase de marquage.

4. Procédé selon la revendication 1, caractérisé en ce que la peroxydase inactivée par l'hydroxyméthylhydroperoxyde est ajoutée en même temps que la peroxydase de marquage.

5. Procédé selon la revendication 1, caractérisé en ce que la peroxydase inactivée par l'hydroxyméthylhydroperoxyde est de nouveau éliminée avant l'addition de la peroxydase de marquage.

6. Procédé selon au moins l'une des revendications 1 à 4, caractérisé en ce que la peroxydase inactivée par l'hydroxyméthylhydroperoxyde est présente à une concentration de 0,002 à 10 mg/ml de mélange d'essai.

7. Procédé selon la revendication 1, caractérisé en ce que la peroxydase inactivée par l'hydroxyméthylhydroperoxyde est présente dans une plage de concentrations allant de 0,02 à 5 mg/ml.

8. Procédé selon la revendication 1, caractérisé en ce que la peroxydase inactivée par l'hydroxyméthylhydroperoxyde est présente dans une plage de concentrations allant de 0,2 à 2 mg/ml.

9. Utilisation de peroxydase inactivée par l'hydroxyméthylhydroperoxyde, dans un procédé de détection immunochimique.

10. Utilisation de peroxydase inactivée par l'hydroxyméthylhydroperoxyde, dans un agent de diagnostic immunochimique pour la détection d'un analyte dans un échantillon d'un matériel biologique se trouvant sous forme liquide.

11. Procédé selon la revendication 2, caractérisé en ce que la phase solide est une plaque de microtitrage.
